# EUROPEAN PATENT SPECIFICATION

(11) **EP 4 327 717 B1**
(45) Date of publication and mention of the grant of the patent: **24.12.2025**
(21) Application number: 22191580.4
(22) Date of filing: 22.08.2022
(51) Int. Cl.: A61B 1/00, G02B 21/16, G02B 21/36

(54) **MEDICAL IMAGING SYSTEM AND METHOD OF OPERATING A MEDICAL IMAGING SYSTEM**
MEDIZINISCHES BILDGEBUNGSSYSTEM UND VERFAHREN ZUM BETRIEB EINES MEDIZINISCHEN BILDGEBUNGSSYSTEMS
SYSTÈME D'IMAGERIE MÉDICALE ET PROCÉDÉ DE FONCTIONNEMENT D'UN SYSTÈME D'IMAGERIE MÉDICALE

(43) Date of publication of application: 28.02.2024
(73) Proprietor: LEICA INSTRUMENTS (SINGAPORE) PTE. LTD., Singapore 618299 (SG)
(72) Inventor: THEMELIS, George, 88131 Lindau (DE)
(74) Representative: Paustian & Partner Patentanwälte mbB

(56) References cited:
- EP-A1- 3 206 061
- US-A1- 2017 154 463
- MACKINNON NICHOLAS ET AL: "Towarddiagnosis of skin cancer using multimode imaging dermoscopy: (I) clinical system development and validation", PROGRESS IN BIOMEDICAL OPTICS AND IMAGING, SPIE - INTERNATIONAL SOCIETY FOR OPTICAL ENGINEERING, BELLINGHAM, WA, US, vol. 8947, 4 March 2014 (2014-03-04), pages 89470I - 89470I, XP060033841, ISSN: 1605-7422, ISBN: 978-1-5106-0027-0, DOI: 10.1117/12.2041818
- KANAMORI KATSUHIRO: "Image enhancement of surface micro-structure on mucosa for polarimetric endoscopy", PROGRESS IN BIOMEDICAL OPTICS AND IMAGING, SPIE - INTERNATIONAL SOCIETY FOR OPTICAL ENGINEERING, BELLINGHAM, WA, US, vol. 9318, 5 March 2015 (2015-03-05), pages 93180O - 93180O, XP060048885, ISSN: 1605-7422, ISBN: 978-1-5106-0027-0, DOI: 10.1117/12.2075787

## Description

### Technical Field

This application relates to a medical imaging system and a method of operating a medical imaging system.

### Background

Medical imaging systems are known in which a tissue is illuminated with visible light, light from the tissue is collected and used to generate a set of image data which can be used to create a visible light image of the tissue. The apparatus for illuminating the object and collecting the light from the tissue may be an apparatus such as a microscope or an endoscope.

In such systems, the light which is collected from the tissue comprises back-scattered light and specular reflections. Specular reflections occur at the interface between the tissue and the air / liquid surrounding the tissue, when a proportion of the incident light is reflected from the tissue surface. These specular reflections can be so bright that they obscure some of the detail in the image of the tissue.

This application relates to ways of operating a medical imaging system which may improve the quality of images obtained where specular reflections are present.

MACKINNON NICHOLAS ET AL: "Towarddiagnosis of skin cancer using multimode imaging dermoscopy: (I) clinical system development and validation", PROGRESS IN BIOMEDICAL OPTICS AND IMAGING, SPIE • INTERNATIONAL SOCIETY FOR OPTICAL ENGINEERING, BELLINGHAM, WA, US, vol. 8947, 4 March 2014 (2014-03-04), pages 894701-894701, XP060033841, ISSN: 1605-7422, DOI: 10.1117/12.2041818 ISBN: 978-1-5106-0027-0, disclose a multimode dermoscope combining fluorescence, polarization, and hyperspectral imaging.EP 3 206 061 A1 is related to an illumination filter system for medical imaging comprising a first optical filter adapted to quench light of at least one fluorescence emission band within the visible spectrum.

KANAMORI KATSUHIRO: "Image enhancement of surface micro-structure on mucosa for polarimetric endoscopy", PROGRESS IN BIOMEDICAL OPTICS AND IMAGING, SPIE - INTERNATIONAL SOCIETY FOR OPTICAL ENGINEERING, BELLINGHAM, WA, US, vol. 9318, 5 March 2015 (2015-03-05), pages 931800-931800, XP060048885, ISSN: 1605-7422, DOI: 10.1117/12.2075787 ISBN: 978-1-5106-0027-0, disclose an image processing method for endoscopy that enhances the appearance of microstructures on mucosa, by employing two pairs of parallel- and crossed-nicols polarimetric images, from which an averaged subtracted polarization image.

US 2017/154463 A1 is related to a method for processing image information, with a step to receive a first two-dimensional data set responsive to a cross-polarized image of a scene, wherein subject matter is illuminated to substantially avoid shadows in the cross-polarized image; and a step to receive a second two-dimensional data set responsive to a co-polarized image of substantially the same scene illuminated to substantially avoid shadows in the co-polarized image; and a step to perform a difference blend operation with the first two-dimensional data set and the second two-dimensional data set to generate an isolated-specular surface texture.

### Summary

The invention is defined by the subject matter of the independent claims. The dependent claims provide information for further embodiments.

According to a first aspect of the disclosed technology we provide a medical imaging system comprising an image data collection apparatus which is configured to collect a first image readout from an object and a second image readout from the object, the first image readout representing back-scattered light and light specular reflections collected from the object, the second image readout representing back-scattered light collected from the object, the imaging system further comprising a processor which is configured to:
a. process the first image readout and second image readout to generate a first image data set which represents light specular reflection image of the object,
b. to use the second image readout to generate a second image data set which represents a back-scattered light image of the object, and
c. to combine the first first image data set with the second image data set to create an enhanced image data set representing an enhanced light image of the object.

The processor is configured to process either or both of the first image data set and the second image data set before combining them to create the enhanced image data set.

The image data collection apparatus may comprise an illumination system which is operable to illuminate the object with polarized light having a polarization axis.

The image data collection apparatus may be configured to collect light from the object which is polarized along a polarization axis which is parallel to the polarization axis of the light from the illumination system to generate the first image readout, and to collect light from the object which is polarized along a polarization axis which is perpendicular to the polarization axis of the illumination system to generate the second image readout.

The image data collection apparatus may further comprise a first light sensor which generates the first image readout and a second light sensor which generates the second image readout.

The image data collection apparatus further comprises a beam splitter which splits the light into a first and second portions, the first light sensor detecting the first light portion and the second light sensor detecting the second light portion.

The image data collection apparatus may further comprise a first polarizer which is arranged in the path of the first light portion and which has a polarization axis which is generally parallel to the polarization axis of the light from the illumination system, and a second polarizer which is arranged in the path of the second light port and which has a polarization axis which is generally perpendicular to the polarization axis of the light from the illumination system.

The image data collection apparatus may further be provided with second beam splitter which is located in the path of second portion of light, and a third light sensor, the second beam splitter being configured to direct light in one or more relatively narrow fluorescence emission bands to the third light sensor and the remaining light to the second light sensor.

According to a second aspect of the disclosed technology, we provide a method of operating a medical imaging system to generate an image of an object, the method comprising:
a) acquiring collecting a first image readout from the object and a second image readout from the object, the first image readout representing back-scattered light and light specular reflections collected from the object, and the second image readout representing back-scattered light collected from the object,
b) processing the first image readout and second image readout to generate a first image data set which represents visible light specular reflection image of the object,
c) using the second image readout to generate a second image data set which represents a back-scattered visible light image of the object, and
d) combining the first processed first image data set with the second image data set respectively to create a enhanced image data set representing an enhanced light image of the object.

The method further comprises processing either or both of the first image data set and the second image data set before combining them to create the enhanced image data set.

According to third aspect of the disclosed technology we provide a computer program product comprising program code stored on a machine-readable medium, the computer program being configured to carry out the method of the second aspect when the computer program runs on a computer system of a medical imaging system.

### Short Description of the Drawings

Embodiments of the invention will now be described with reference to the accompanying figures of which
FIGURE 1 shows a schematic illustration of medical imaging system comprising an image data collection apparatus and a computer system,
FIGURE 2 shows a schematic illustration of the image data collection apparatus of the medical imaging system illustrated in Figure 1,
FIGURE 3 shows a schematic illustration of the illumination system and light collection system of the image data collection apparatus illustrated in Figure 2,
FIGURE 4 shows a schematic illustration of the light sensors of the light collection system illustrated in Figure 3 and the computer system of the medical imaging system illustrated in Figure 1,
FIGURE 5 shows a schematic illustration of an alternative embodiment of image data collection apparatus suitable for use in the medical imaging system illustrated in Figure 1,
FIGURE 6 shows a schematic illustration of a further alternative embodiment of image data collection apparatus suitable for use in the medical imaging system illustrated in Figure 1,
FIGURE 7 shows a schematic illustration of a further alternative embodiment of image data collection apparatus suitable for use in the medical imaging system illustrated in Figure 1,
FIGURE 8 shows a schematic illustration of a method of generating an enhanced image using image data collected using the any of the image data collection apparatus illustrated in Figures 2, 5, 6, or 7,
FIGURE 9 shows a schematic illustration of an alternative method of generating an enhanced image using image data collected using the any of the image data collection apparatus illustrated in Figures 2, 5, 6, or 7,
FIGURE 10 shows a schematic illustration of a a further alternative method of generating an enhanced image using image data collected using the any of the image data collection apparatus illustrated in Figures 2, 5, 6, or 7,
FIGURE 11 shows a schematic illustration of the alternative embodiment of image data collection apparatus suitable for use in the medical imaging system illustrated in Figure 1,
FIGURE 12 shows a schematic illustration of the illumination system and light collection system of the alternative embodiment of image data collection apparatus illustrated in Figure 11,
FIGURE 13 shows a schematic illustration of the light sensors of the light collection system illustrated in Figure 12 and the computer system of the medical imaging system illustrated in Figure 1,
FIGURE 14 shows a schematic illustration of a method of generating an enhanced image using image data collected using the image data collection apparatus illustrated in Figure 11,
   and
FIGURE 15 shows a schematic illustration of an alternative method of generating an enhanced image using image data collected using the image data collection apparatus illustrated in Figure 11.

### Detailed Description

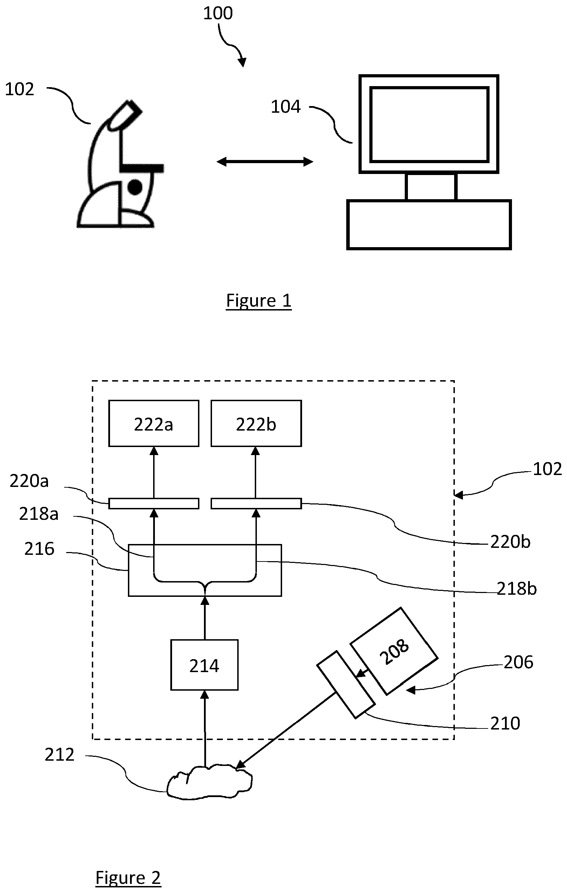

Referring now to Figure 1, there is shown a medical imaging system 100 comprising an image data collection apparatus 102 and a computer system 104. The image data collection apparatus 102 is configured to generate image data and is connected to the computer system 104 in such a way that image data generated by the image data collection apparatus can be transmitted to and processed by the computer system 104.

In relation to the terms "image" and "image data" or operations carried out of images or image data mentioned in the following, it should be understood that the terms "image" and "image data" can be used synonymously, as the image represents the totality of the image data making up the image. The image data may be grouped into pixels, and a "pixel" corresponds to an ordered grouping of the image data. The images and image data are processed and/or stored digitally and are themselves digital.

The image data collection apparatus 102 may be an apparatus such as a microscope or endoscope. In the embodiments described below the image data collection apparatus 102 is a microscope, and will therefore hereinafter be referred to as microscope 102. It should be appreciated, however, that this need not be the case. Any other suitable image data collection apparatus 102 could be used in place of a microscope. It should also be appreciated that the image data collection apparatus 102 could be a stereoscopic microscope, in which case, method described below could be replicated for each stereoscopic channel of the microscope 102.

The microscope 102 is illustrated schematically in more detail in Figure 2, and comprises an illumination system 206 which comprises a light source 208 and a linear polarizer 210, and is arranged to illuminate an object 212. In this embodiment, the light source 208 produces light across the entire visible light spectrum, i.e. "white light", and may be a xenon lamp. The linear polarizer 210 is arranged to polarise the light from the light source 208 so that all the light incident on the object 212 is polarised along a single axis. The illumination system 206 may additionally include one or more filters, for example to filter out any non-visible spectrum light produced by the light source 208.

The microscope also includes an optics system 214 which comprises a set of conventional microscope lenses which collect light from the object and direct the collected light to a beam splitter 216 which splits the collected light into two substantially equal portions, and directs a first portion of the light along a first light path 218a to first linear polarizer 220a, and a second portion of the light along a second light path 218b to a second linear polarizer 220b. The microscope is also provided with two light sensors, which are typically visible light cameras such as a CCD camera. The light sensors could equally detect invisible light, such as ultraviolet or infra-red light.

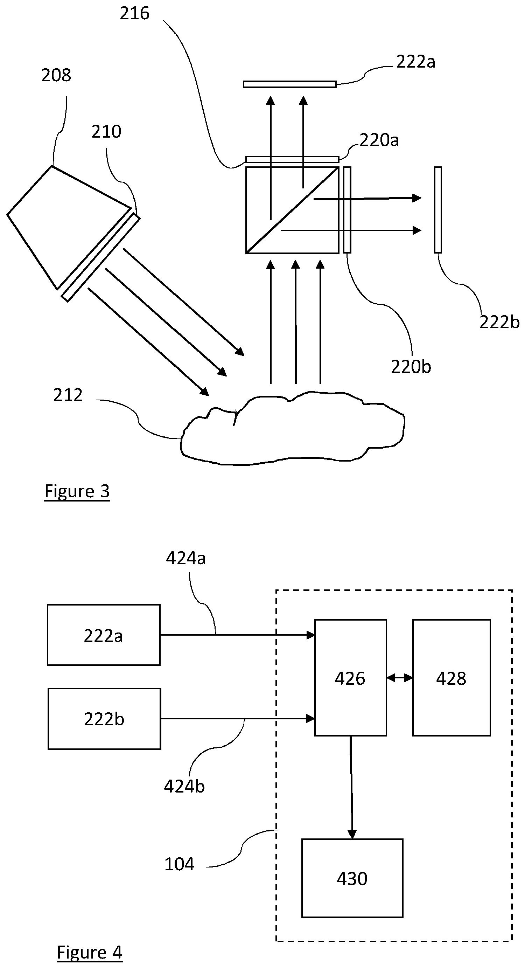

Light from the first linear polarizer 220a falls on the first light sensor 222a, and the light from the second linear polarizer 222b falls on the second light sensor 222b. This is also illustrated schematically in Figure 3.

The first linear polarizer 220a is oriented so that the polarization axis is aligned with the polarization axis of the liner polariser 210 in the illumination system 206. The light collected by the first light sensor 222a is thus a combination of specular reflections and back-scattered light from the object. The second linear polarizer 220b is oriented so that its polarization axis is perpendicular to the polarization axis of the first linear polarizer 220a. The light collected by the second light sensor 222b thus contains only back-scattered light from the object 212.

The first and second sensors 222a, 222b are connected to a processor 426 of the computer system 104 via a first signal line 424a and a second signal line 424b by means of which digital image data may be transmitted from the sensors 222a, 222b to the processor 426, as illustrated in Figure 4. A first image readout IR₁, which contains the image data of the light captured by the first light sensor 222a is sent to the processor 426 via the first signal line 424a, and a second image readout IR₂, which contains the image data of the light captured by the second light sensor 222b is sent to the processor 426 via the second signal line 424b.

The processor 426 is configured to process the first and second image readouts IR₁, IR₂ and to produce an enhanced image data set which may be used to generate an enhanced image of the object 212, as will be explained further below. The computer system 104 in this embodiment comprises a display 430 on which the enhanced image may be displayed, and a memory 428 in which the enhanced image data set may be stored.

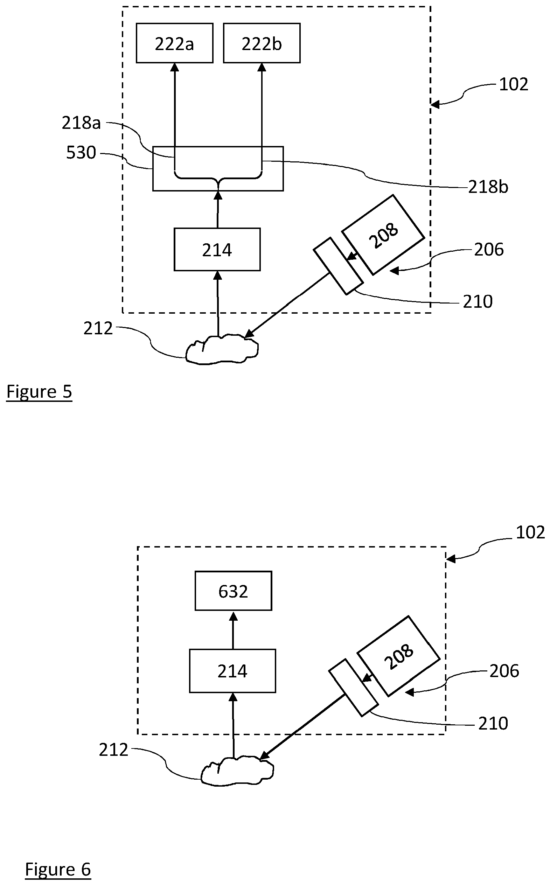

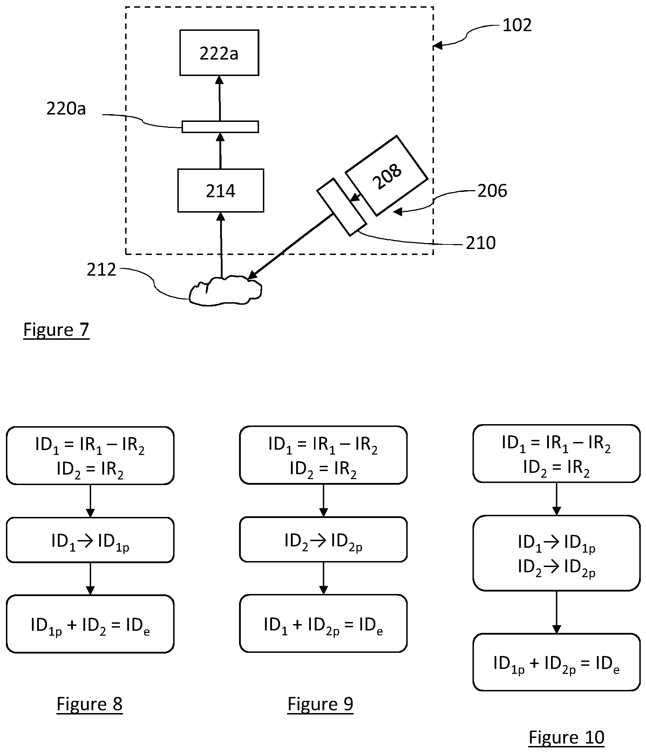

It should be appreciated that first and second image readouts IR₁, IR₂ represent light collected from the object split based on the polarization of the light. Whilst in this embodiment, this is achieved using a beam splitter 216 and two orthogonal linear polarizers 220a, 220b, this need not be the case. For example, the beam splitter 216 and linear polarizers 220a, 220b may be replaced with a polarization beam splitter 530, which splits the light based on its polarization. The first and second linear polarizers may therefore be omitted as illustrated in Figure 5. Alternatively, the beam splitter 216 may be omitted entirely, and an imaging sensor with polarization resolution 632, which has polarizers on each pixel, and is thus able to capture simultaneously two or more images with different polarizations (such at the POLARSENS^{™} CMOS image sensor produced by Sony Semiconductor Solutions Corp.), may be used in place of the two light sensors, as illustrated in Figure 6. In a further alternative embodiment, illustrated in Figure 7, the beam splitter 216, second linear polarizer 220b and second light sensor 220b may be omitted, and the remaining light sensor 220a used to carry out sequential imaging in which light sensor 220a is used to obtain the first image readout I₁, the first linear polarizer 220a rotated through 90°, and the light sensor 220a used obtain the second image readout I₂. This process is then repeated for every image, the orientation of the linear polarizer 220a being rotated through 90° after each image readout is captured.

The processor 426 is configured to process the first and second image readouts IR₁, IR₂ to create a first image data set ID₁ representing the specular reflections from the object 212, and a second image data set ID₂ representing the back-scattering light from the object 212. In this embodiment, as the second image readout IR₂ is derived from light which passes through a linear polarizer 220b with a polarization axis which is perpendicular to the polarization axis of the source polarizer 210, the second image data set ID₂ is the second image readout IR₂. The first image data set ID₁ is obtained by subtracting the second image readout IR₂ from the first image readout IR₁.

The processor 426 is also configured to combine the first image data set ID₁ with the second image data set ID₂ to generate an enhanced image data set IDe which represents an enhanced light image of the obect 212. Whilst not essential, in this embodiment, the processor 426 is also configured to process one or both of the first and second image data sets ID₁, ID₂, before recombining the image data sets to generate an enhanced image data set IDₑ, from which an enhanced image of the object may be created. The image data sets may be recombined by added the two image data sets together, by making a linear combination of the two image data sets, or by using alpha blending on the second image data set so that the second image data set does not hide the colour in the first image data date ID₁, or for each pixel, setting each pixel in the enhanced image data set IDₑ at the colour and brightness level of the brightest of the two corresponding pixels of each image data sets.

In one embodiment, the processor 426 is configured to process the first image data set ID₁ to create a processed first image data set ID₁ₚ, and then to combine the processed first image data set ID₁ₚ with the second image data set ID₂ to generate the enhanced image data set IDₑ. The first image data set ID₁ could, for example, be processed by applying a threshold to remove weaker reflections from the image data set, by applying a gamma filter, or by applying a spatial filter that attenuates the core of a reflection spot while keeping the edges intense, so that the reflection spot is semi-transparent but the edges are clearly seen. This method is illustrated schematically in Figure 8.

In one embodiment, the processor 426 is configured to process the second image data set ID₁ to create a processed second image data set ID₂ₚ, and then to combine the processed second image data set ID₂ₚ with the first image data set ID₁ to generate the enhanced image data set IDₑ. The second image data set ID₁ could, for example, be processedby applying a gamma filter, by applying a contrast enhancement filter, or by applying other standard image processing filter or algorithm. This method is illustrated schematically in Figure 9.

The processor 426 could equally be configured to process both the first and second image data sets ID₁, ID₂ before combining the first and second processed image data sets ID₁ₚ, ID₂ₚ, to generate the enhanced image data set IDₑ, as is illustrated schematically in Figure 10.

In one embodiment, the second image data ID₂ is processed using a technique known as multispectral fluorescence imaging as described in EP 3 206 061, for example. In this technique image data, both visible light and fluoresence light in one or more narrow fluorescence bands emitted from the object are collected simultaneously, to generate fluorence image data representing a fluorescent light image of the object and visible light image data representing a visible light image of the object. A colour not usually present in the object (for example green if the object is human tissue) is ascribed to each fluorescence band, and then the fluorescent light image data used to modify the visible light image data so that the fluorescent light emitted by the object is shown in the selected colour in the visible light image.

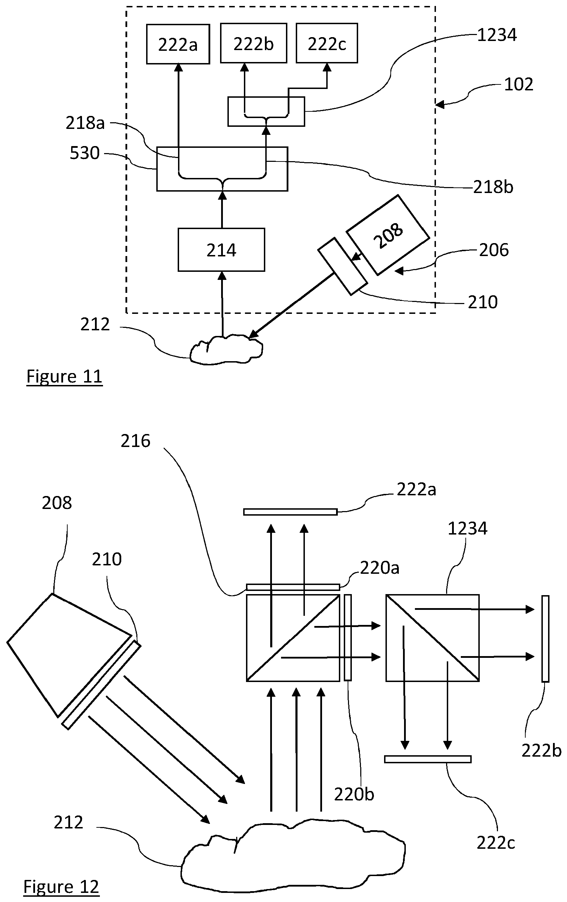

To do this, the medical imaging system 100 is further provided with second beam splitter 1234 which is placed in the path of light passing from the second polarisr 220b, and an additional (third) light sensor 222c is provided, the third light sensor 222c also being connected to the processor via a third signal line 424c. The second beam splitter 1234 thus splits the back-scattered light from the object 212, and is configured to direct light in one or more relatively narrow fluorescence emission bands to the third light sensor 222c and the remaining light to the second light sensor 222b. This is illustrated schematically in Figures 11 and 12.

The narrow fluorescence emission bands could correspond to the wavelength(s) of fluorescence light emitted by one or more fluorophores present in the object 212, or if no fluorophore is used, to one or more pre-selected wavelengths or narrow wavelength bands. For example, the beam splitter 1234 may be a dichroic beam splitter which is configured to separate out light in one band in the visible red spectrum corresponding to 5-aminolevulinic acid (5-ALA) emission, and light in a second band in the infra-red spectrum corresponding to indocyanine green (ICG) emission. The third light sensor 222c will be configured to detect light of wavelengths appropriate to the wavelengths of the light separated out by the second beam splitter 1234. For example, if the fluorescence bands are in the near infra-red (NIR) range, the third light sensor 222c may be a NIR camera.

Filters may be placed in the light path between the second beam splitter 1234 and the second and third light sensors 222b, 222c as described in EP 3 206 061, for example. A band stop filter may be arranged to filter one portion of the light exiting the beam splitter, before it reaches the second light sensor 222b, and a band pass filts may be arranged to filter the other portion of light from the second beam splitter 1234 before it reaches the third light sensor 222c.

A second image readout IR₂, which contains the image data of the light captured by the second light sensor 222b and a third image readout IR₃, which contains the image data of the light captured by the third light sensor 222c are sent ot the processor 426 via the second and third signal lines 424b, 424c. These image readouts IR₂, IR₃ are processed by the processor 426 to produce the processed second image data set ID₂ₚ. This could be done by assigning a colour (such as green) to the light collected by the third light sensor 222c and adding that colour to the colour of the light collected by the second light sensor 222b on a pixel by pixel basis, or using alpha-blending to mix the images. Further details of how the light collected by the second and third light sensors 222b, 222c could be processed to generate the processed second image data set ID₂ₚ can be found in EP 3 206 061.

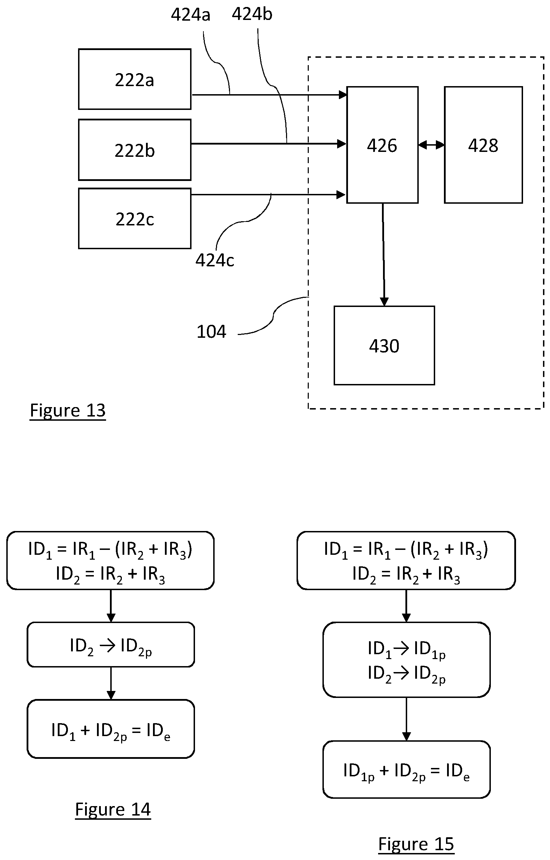

In this embodiment, the first image data set ID₁, which represents the specular reflections from the object 212, is obtained by subtracting both the second and third image readouts IR₂, IR₃ from the first image readout IR₁. The first image data set ID₁ can be left unprocessed, or processed to generate a processed first image data set ID₁ₚ as described above. The processor 426 then combines the processed second image data set ID₂ₚ with either the first image data set ID₁ or the processed first image data set ID1p as described above to form the enhanced image data set IDₑ. This is illustrated schematically in Figures 14 and 15.

Whilst in the embodiments described above, the object 212 is illuminated with linearly polarized light, the invention could equally be implemented using circularly polarized light instead.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to a microscope comprising a system as described in connection with one or more of the Figs. 1 to 12. Alternatively, a microscope may be part of or connected to a system as described in connection with one or more of the Figs. 1 to 12.

Figure 1 shows a schematic illustration of a medical imaging system configured to perform a method described herein. The system 100 comprises a microscope 102 and a computer system 104. The computer system 104 is configured to execute at least a part of a method described herein. The computer system 104 may be configured to execute a machine learning algorithm.

The computer system 104 and microscope 102 may be separate entities but can also be integrated together in one common housing. The computer system 104 may be part of a central processing system of the microscope 102 and/or the computer system 104 may be part of a subcomponent of the microscope 102, such as a sensor, an actor, a camera or an illumination unit, etc. of the microscope 102.

The computer system 104 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 104 may comprise any circuit or combination of circuits. In one embodiment, the computer system 104 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 104 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 104 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 104 may also include a display device, one or more speakers, and a keyboard and/or controller, which can include a mouse, trackball, touch screen, voice-recognition device, or any other device that permits a system user to input information into and receive information from the computer system 104.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### List of Reference Signs

- 100 -: medical imaging system
- 102 -: image data collection apparatus
- 104 -: computer system
- 206 -: illumination system
- 208 -: light source
- 210 -: linear polarizer
- 212 -: object
- 214 -: optics system
- 216 -: beam splitter
- 218a -: first light path
- 218b -: second light path
- 220a -: first linear polarizer
- 220b -: second linear polarizer
- 222a -: first light sensor
- 222b -: second light sensor
- 222c -: third light sensor
- 424a -: first signal line
- 424b -: second signal line
- 424c -: third signal line
- 426 -: processor
- 428 -: memory
- 430 -: display632 - imaging sensor
- IR₁ -: first image readout
- IR₂ -: second image readout
- IR₃ -: third image readout
- ID₁ -: first image data set
- ID₂ -: second image data set
- IDₑ -: enhanced image data set
- ID₁ₚ -: first processed image data set
- ID₂, -: second processed image data set
- 1234 -: second beam splitter

## Claims

1. A medical imaging system (100) comprising an image data collection apparatus (102) which is configured to collect a first image readout (IR₁) from an object (212) and a second image readout (IR₂) from the object (212), the first image readout (IR₁) representing back-scattered light and light specular reflections collected from the object (212), the second image readout (IR₂) representing back-scatter light collected from the object (212), the imaging system further comprising a processor (426) which is configured to:
a) process the first image readout (IR₁) and second image readout (IR₂) to generate a first image data set (ID₁) which represents a light specular reflection image of the object (212),
b) to use the second image readout (IR₂) to generate a second image data set (ID₂) which represents a back-scattered light image of the object (212), and
c) to combine the first image data set (ID₁) with the second image data set (ID₂) to create an enhanced image data set (IDₑ) representing an enhanced light image of the object (212);
wherein the processor is configured to process either or both of the first image data set (ID₁) and the second image data set (ID₂) before combining them to create the enhanced image data set (IDₑ); and
wherein the processor is configured to combine two data sets (ID₁, ID₂, ID₁, ID₂ₚ) linearly.

2. A medical imaging system (100) according to claim 1 wherein the image data collection apparatus comprises an illumination system 206 which is operable to illuminate the object (212) with polarized light having a polarization axis.

3. A medical imaging system (100) according to claim 2 wherein the image data collection apparatus (102) is configured to collect light from the object (212) which is polarized along a polarization axis which is parallel to the polarization axis of the light from the illumination system to generate the first image readout IR₁, and to collect light from the object (212) which is polarized along a polarization axis which is perpendicular to the polarization axis of the illumination system to generate the second image readout (IR₂).

4. A medical imaging system according to claim 3 wherein the image data collection apparatus (102) further comprises a first light sensor (222a) which generates the first image readout (IR₁) and a second light sensor (222b) which generates the second image readout (IR₂).

5. A medical imaging system (100) according to claim 4 wherein the image data collection apparatus (102) further comprises a beam splitter (216) which splits the light into a first and second portions, the first light sensor (222a) detecting the first light portion and the second light sensor (222b) detecting the second light portion.

6. A medical imaging system (100) according to claim 5 wherein the image data collection apparatus (102) further comprises a first polarizer (220a) which is arranged in the path of the first light portion and which has a polarization axis which is generally parallel to the polarization axis of the light from the illumination system (206), and a second polarizer (220b) which is arranged in the path of the second light port and which has a polarization axis which is generally perpendicular to the polarization axis of the light from the illumination system (206).

7. A medical imaging system (100) according any one of claims 6 wherein the image data collection apparatus is further provided with second beam splitter (1234) which is located in the path of second portion of light, and a third light sensor 222c , the second beam splitter 1234 being configured to direct light in one or more relatively narrow fluorescence emission bands to the third light sensor 222c and the remaining light to the second light sensor 222b.

8. A method of operating a medical imaging system (100) to generate an image of an object (212), the method comprising:
a) acquiring collecting a first image readout (IR₁) from the object (212) and a second image readout (IR₂) from the object (212), the first image readout (IR₁) representing back-scattered light and light specular reflections collected from the object (212), and the second image readout (IR2) representing back-scatter light collected from the object (212),
b) processing the first image readout (IR₁) and second image readout (IR₂) to generate a first image data set (ID₁) which represents light specular reflection image of the object (212),
c) using the second image readout (IR₂) to generate a second image data set (ID₂) which represents a back-scattered light image of the object (212), and
d) combining the first image data set (ID₁) with the second image data set (ID₂) respectively to create a enhanced image data set (IDₑ) representing an enhanced light image of the object (212);
wherein the method further comprises processing either or both of the first image data set (ID₁) and the second image data set (ID₂) before combining them to create the enhanced image data set (IDₑ); and
wherein the two data sets (ID₁, ID₂, ID₁ₚ ID₂ₚ) are combined linearly.

9. A computer program product comprising program code stored on a machine-readable medium, the computer program being configured to carry out the method of claim 8 when the computer program runs on a computer system of a medical imaging system.

## Patentansprüche

1. Medizinisches Bildgebungssystem (100), umfassend eine Bilddatensammeleinrichtung (102), die konfiguriert ist, um eine erste Bildauslesung (IR₁) von einem Objekt (212) und eine zweite Bildauslesung (IR₂) von dem Objekt (212) zu sammeln, wobei die erste Bildauslesung (IR₁) rückgestreutes Licht und von dem Objekt (212) gesammelte spiegelnde Lichtreflexionen darstellt, wobei die zweite Bildauslesung (IR₂) das von dem Objekt (212) gesammelte Rückstreulicht darstellt, wobei das Bildgebungssystem weiter einen Prozessor (426) umfasst, der konfiguriert ist zum:
a) Verarbeiten der ersten Bildauslesung (IR₁) und der zweiten Bildauslesung (IR₂), um einen ersten Bilddatensatz (ID₁) zu erzeugen, der ein spiegelndes Lichtreflexionsbild des Objekts (212) darstellt,
b) Verwenden der zweiten Bildauslesung (IR₂), um einen zweiten Bilddatensatz (ID₂) zu erzeugen, der ein rückgestreutes Lichtbild des Objekts (212) darstellt, und
c) Kombinieren des ersten Bilddatensatzes (ID₁) mit dem zweiten Bilddatensatz (ID₂), um einen erweiterten Bilddatensatz (IDₑ) zu erzeugen, der ein erweitertes Lichtbild des Objekts (212) darstellt;
wobei der Prozessor konfiguriert ist, um entweder den ersten Bilddatensatz (ID₁) oder den zweiten Bilddatensatz (ID₂) oder beide zu verarbeiten, bevor er sie kombiniert, um den erweiterten Bilddatensatz (IDₑ) zu erzeugen; und
wobei der Prozessor konfiguriert ist, um zwei Datensätze (ID₁, ID₂, ID₁ₚ ID₂ₚ) linear zu kombinieren.

2. Medizinisches Bildgebungssystem (100) nach Anspruch 1, wobei die Bilddatensammeleinrichtung ein Beleuchtungssystem 206 umfasst, das betrieben werden kann, um das Objekt (212) mit polarisiertem Licht, das eine Polarisationsachse aufweist, zu beleuchten.

3. Medizinisches Bildgebungssystem (100) nach Anspruch 2, wobei die Bilddatensammeleinrichtung (102) konfiguriert ist, um Licht von dem Objekt (212) zu sammeln, das entlang einer Polarisationsachse polarisiert ist, die parallel zu der Polarisationsachse des Lichts von dem Beleuchtungssystem ist, um die erste Bildauslesung IR₁ zu erzeugen, und um Licht von dem Objekt (212) zu sammeln, das entlang einer Polarisationsachse polarisiert ist, die senkrecht zur Polarisationsachse des Beleuchtungssystems ist, um die zweite Bildauslesung (IR₂) zu erzeugen.

4. Medizinisches Bildgebungssystem nach Anspruch 3, wobei die Bilddatensammeleinrichtung (102) weiter einen ersten Lichtsensor (222a), der die erste Bildauslesung (IR₁) erzeugt, und einen zweiten Lichtsensor (222b), der die zweite Bildauslesung (IR₂) erzeugt, umfasst.

5. Medizinisches Bildgebungssystem (100) nach Anspruch 4, wobei die Bilddatensammeleinrichtung (102) weiter einen Strahlteiler (216) umfasst, der das Licht in einen ersten und einen zweiten Abschnitt teilt, wobei der erste Lichtsensor (222a) den ersten Lichtabschnitt detektiert und der zweite Lichtsensor (222b) den zweiten Lichtabschnitt detektiert.

6. Medizinisches Bildgebungssystem (100) nach Anspruch 5, wobei die Bilddatensammeleinrichtung (102) weiter einen ersten Polarisator (220a) umfasst, der im Pfad des ersten Lichtabschnitts angeordnet ist und der eine Polarisationsachse aufweist, die im Allgemeinen parallel zur Polarisationsachse des Lichts vom Beleuchtungssystem (206) ist, und einen zweiten Polarisator (220b), der im Pfad des zweiten Lichtanschlusses angeordnet ist und der eine Polarisationsachse aufweist, die im Allgemeinen senkrecht zur Polarisationsachse des Lichts aus dem Beleuchtungssystem (206) ist.

7. Medizinisches Bildgebungssystem (100) nach einem der Ansprüche 6, wobei die Bilddatensammeleinrichtung weiter mit einem zweiten Strahlteiler (1234), der im Pfad des zweiten Lichtabschnitts lokalisiert ist, und einem dritten Lichtsensor 222c bereitgestellt ist, wobei der zweite Strahlteiler 1234 konfiguriert ist, um Licht in einem oder mehreren relativ schmalen Fluoreszenz-Emissionsbändern auf den dritten Lichtsensor 222c und das restliche Licht auf den zweiten Lichtsensor 222b zu richten.

8. Verfahren zum Betreiben eines medizinischen Bildgebungssystems (100) zum Erzeugen eines Bildes eines Objekts (212), wobei das Verfahren Folgendes umfasst:
a) Erfassen eines Sammelns einer ersten Bildauslesung (IR₁) von dem Objekt (212) und einer zweiten Bildauslesung (IR₂) von dem Objekt (212), wobei die erste Bildauslesung (IR₁) zurückgestreutes Licht und vom Objekt (212) gesammelte spiegelnde Lichtreflexionen darstellt, und die zweite Bildauslesung (IR2) das vom Objekt (212) gesammelte Rückstreulicht darstellt,
b) Verarbeiten der ersten Bildauslesung (IR₁) und der zweiten Bildauslesung (IR₂), um einen ersten Bilddatensatz (ID₁) zu erzeugen, der ein spiegelndes Lichtreflexionsbild des Objekts (212) darstellt,
c) Verwenden der zweiten Bildauslesung (IR₂), um einen zweiten Bilddatensatz (ID₂) zu erzeugen, der ein rückgestreutes Lichtbild des Objekts (212) darstellt, und
d) Kombinieren des ersten Bilddatensatzes (ID₁) mit dem zweiten Bilddatensatz (ID₂), um jeweils einen erweiterten Bilddatensatz (IDₑ) zu erzeugen, der ein erweitertes Lichtbild des Objekts (212) darstellt;
wobei das Verfahren weiter Verarbeiten eines oder beider des ersten Bilddatensatzes (ID₁) und des zweiten Bilddatensatzes (ID₂)umfasst, bevor diese kombiniert werden, um den erweiterten Bilddatensatz (IDₑ) zu erzeugen; und
wobei die beiden Datensätze (ID₁, ID₂, ID₁ₚ ID₂ₚ) linear kombiniert werden.

9. Computerprogrammprodukt, umfassend einen auf einem maschinenlesbaren Medium gespeicherten Programmcode, wobei das Computerprogramm konfiguriert ist, um das Verfahren nach Anspruch 8 auszuführen, wenn das Computerprogramm auf einem Computersystem eines medizinischen Bildgebungssystems läuft.

## Revendications

1. Système d'imagerie médicale (100) comprenant un appareil (102) de collecte de données d'image qui est configuré pour collecter une première lecture (IR₁) d'image d'un objet (212) et une seconde lecture (IR₂) d'image de l'objet (212), la première lecture (IR₁) d'image représentant la lumière rétrodiffusée et les réflexions spéculaires de lumière collectées de l'objet (212), la seconde lecture (IR₂) d'image représentant la lumière rétrodiffusée collectée de l'objet (212), le système d'imagerie comprenant en outre un processeur (426) qui est configuré pour :
a) traiter la première lecture (IR₁) d'image et la seconde lecture (IR₂) d'image pour générer un premier ensemble de données (ID₁) d'image qui représente une image de réflexion spéculaire de lumière de l'objet (212),
b) utiliser la seconde lecture (IR₂) d'image pour générer un second ensemble de données (ID₂) d'image qui représente une image de lumière rétrodiffusée de l'objet (212), et
c) combiner le premier ensemble de données (ID₁) d'image avec le second ensemble de données (ID₂) d'image pour créer un ensemble de données (IDₑ) d'image améliorée représentant une image de lumière améliorée de l'objet (212) ; dans lequel le processeur est configuré pour traiter l'un ou l'autre ou les deux du premier ensemble de données (ID₁) d'image et du second ensemble de données (ID₂) d'image avant de les combiner pour créer l'ensemble de données (IDₑ) d'image améliorée ; et
dans lequel le processeur est configuré pour combiner de manière linéaire deux ensembles de données (ID₁, ID₂, ID₁ₚ, ID₂ₚ).

2. Système d'imagerie médicale (100) selon la revendication 1 dans lequel l'appareil de collecte de données d'image comprend un système d'éclairage (206) qui peut être utilisé pour éclairer l'objet (212) avec une lumière polarisée présentant un axe de polarisation.

3. Système d'imagerie médicale (100) selon la revendication 2 dans lequel l'appareil (102) de collecte de données d'image est configuré pour collecter la lumière de l'objet (212) qui est polarisée le long d'un axe de polarisation qui est parallèle à l'axe de polarisation de la lumière provenant du système d'éclairage pour générer la première lecture (IR₁) d'image, et pour collecter la lumière de l'objet (212) qui est polarisée le long d'un axe de polarisation qui est perpendiculaire à l'axe de polarisation du système d'éclairage pour générer la seconde lecture (IR₂) d'image.

4. Système d'imagerie médicale selon la revendication 3 dans lequel l'appareil (102) de collecte de données d'image comprend en outre un premier capteur (222a) de lumière qui génère la première lecture (IR₁) d'image et un deuxième capteur (222b) de lumière qui génère la seconde lecture (IR₂) d'image.

5. Système d'imagerie médicale (100) selon la revendication 4 dans lequel l'appareil (102) de collecte de données d'image comprend en outre un séparateur (216) de faisceau qui divise la lumière en une première et seconde portion, le premier capteur (222a) de lumière détectant la première portion de lumière et le deuxième capteur (222b) de lumière détectant la seconde portion de lumière.

6. Système d'imagerie médicale (100) selon la revendication 5 dans lequel l'appareil (102) de collecte de données d'image comprend en outre un premier polariseur (220a) qui est agencé dans le trajet de la première portion de lumière et qui présente un axe de polarisation qui est généralement parallèle à l'axe de polarisation de la lumière provenant du système d'éclairage (206), et un second polariseur (220b) qui est agencé dans le trajet du second orifice de lumière et qui présente un axe de polarisation qui est généralement perpendiculaire à l'axe de polarisation de la lumière provenant du système d'éclairage (206).

7. Système d'imagerie médicale (100) selon l'une quelconque des revendications 6 dans lequel l'appareil de collecte de données d'image est en outre pourvu d'un second séparateur (1234) de faisceau qui est situé dans le trajet de la seconde portion de lumière, et d'un troisième capteur (222c) de lumière, le second séparateur (1234) de faisceau étant configuré pour diriger la lumière dans une ou plusieurs bandes d'émission de fluorescence relativement étroites vers le troisième capteur (222c) de lumière et la lumière restante vers le deuxième capteur (222b) de lumière.

8. Procédé de fonctionnement d'un système d'imagerie médicale (100) pour générer une image d'un objet (212), le procédé comprenant :
a) l'acquisition de la collecte d'une première lecture (IR₁) d'image de l'objet (212) et d'une seconde lecture (IR₂) d'image de l'objet (212), la première lecture (IR₁) d'image représentant la lumière rétrodiffusée et les réflexions spéculaires de lumière collectées de l'objet (212), et la seconde lecture (IR₂) d'image représentant la lumière rétrodiffusée collectée de l'objet (212),
b) le traitement de la première lecture (IR₁) d'image et de la seconde lecture (IR₂) d'image pour générer un premier ensemble de données (ID₁) d'image qui représente l'image de réflexion spéculaire de lumière de l'objet (212),
c) l'utilisation de la seconde lecture (IR₂) d'image pour générer un second ensemble de données (ID₂) d'image qui représente une image de lumière rétrodiffusée de l'objet (212), et
d) la combinaison du premier ensemble de données (ID₁) d'image avec le second ensemble de données (ID₂) d'image respectivement pour créer un ensemble de données (IDₑ) d'image améliorée représentant une image de lumière améliorée de l'objet (212) ;
dans lequel le procédé comprend en outre le traitement de l'un ou l'autre ou des deux du premier ensemble de données (ID₁) d'image et du second ensemble de données (ID₂) d'image avant de les combiner pour créer l'ensemble de données (IDₑ) d'image améliorée ; et
dans lequel les deux ensembles de données (ID₁, ID₂, ID₁ₚ, ID₂ₚ) sont combinés de manière linéaire.

9. Produit programme d'ordinateur comprenant un code de programme stocké sur un support lisible par machine, le programme d'ordinateur étant configuré pour mettre en œuvre le procédé selon la revendication 8 lorsque le programme d'ordinateur est exécuté sur un système informatique d'un système d'imagerie médicale.
